(19) Europäisches Patentamt
European Patent Office
Office européen des brevets

(11) Publication number: 0 185 381 B1

# (12) EUROPEAN PATENT SPECIFICATION

(45) Date of publication of patent specification: 26.07.89

(51) Int. Cl.$^4$: C 07 D 409/06, A 61 K 31/41, A 61 K 31/415

(21) Application number: 85116287.5

(22) Date of filing: 19.12.85

## (54) 2-Azolylmethyl-3-difluorobenzyloxy-2,3-dihydrofluorobenzo(b)thiophenes, process for their preparation and compositions containing them.

(30) Priority: 21.12.84 US 684872

(43) Date of publication of application: 25.06.86 Bulletin 86/26

(45) Publication of the grant of the patent: 26.07.89 Bulletin 89/30

(84) Designated Contracting States: AT BE CH DE FR GB IT LI LU NL SE

(58) References cited: EP-A-0 054 233

(73) Proprietor: SCHERING CORPORATION
2000 Galloping Hill Road
Kenilworth New Jersey 07033 (US)

(72) Inventor: Rane, Dinanath Fakir
41 Lantern Lane
Sayreville New Jersey 08872 (US)
Inventor: Desai, Jagdish Armitlal
2305 Westminster Boulevard
Parlin New Jersey 08859 (US)
Inventor: Pike, Russell Edwin
RD No. 1, 31 Florence Street
Stanhope New Jersey 07874 (US)

(74) Representative: von Kreisler, Alek, Dipl.-Chem. et al
Deichmannhaus am Hauptbahnhof
D-5000 Köln 1 (DE)

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 185 381 B1

Courier Press, Leamington Spa, England.

**Description**

This invention relates to 2-azolylmethyl-3-difluorobenzyloxy-2,3-dihydro-5-fluorobenzo[b]thiophenes, antifungal compounds, and pharmaceutical compositions containing them.

U.S. Patent 4,352,808 (Rane et al.) and the corresponding European patent application EP 54,233 disclose 3-aralkyloxy-2,3-dihydro-2-(1H-imidazolylmethyl)benzo[b]thiophene antifungal compounds.

U.S. Patent 4,431,816 (Rane et al.) discloses 3-hydroxy-2,3-dihydro-2-(1H-imidazolylmethyl)benzo[b]-thiophene antifungal compounds. U.S. Patent 4,468,404 discloses 3-arylalkoxy-2,3-dihydro-2-(1H-1,2,4-triazolylmethyl)benzo[b]thiophene antifungal compounds. While U.S. Patent Nos. 4,352,808 and 4,468,404 disclose antifungal compounds that are generic to the compounds of this invention, there is no specific disclosure of the compounds of this invention and no indication that the trifluoro compounds of this invention would be expected to have properties superior compared to the trichloro compounds, e.g., *cis*-5-chloro-3-(2′,6′-dichlorobenzyloxy)-2,3-dihydro-2-(1H-imidazolylmethyl)benzo[b]thiophene specifically disclosed in the above-identified U.S. Patents.

The compounds of this invention are represented by the following formula I

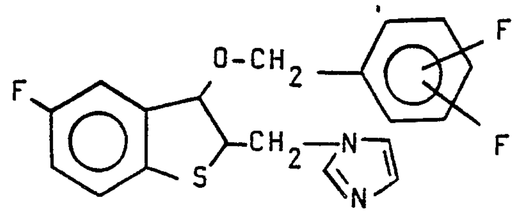

I

or a pharmaceutically acceptable acid addition salt thereof, wherein the fluoro-substituents are in positions 2′ and 4′ or 2′ and 6′ in racemic or optically active form.

The present invention also provides a pharmaceutical composition comprising an antifungally effective amount of a compound represented by formula I or a pharmaceutically acceptable acid salt thereof, together with a pharmaceutically acceptable carrier or diluent.

The most preferred compound of the present invention is cis-2-(1H-imidazolylmethyl)-3-(2′,6′-difluorobenzyloxy)-2,3-dihydro-5-fluorobenzo[b]thiophene or a pharmaceutically acceptable acid salt thereof, e.g. HCl.

The preferred pharmaceutical composition of the present invention comprises an antifungally effective amount of *cis*-2-(1H-imidazolylmethyl)-3-(2′,6′-difuorobenzyloxy-2,3-dihydro-5-fluorobenzo[b]thiophene or a pharmaceutically acceptable acid salt thereof together with a pharmaceutically acceptable carrier or diluent.

As used in the specification, the term "$C_1$—$C_6$-alkyl" refers to straight and branched chain alkyl groups of 1 to 6 carbon atoms, such as methyl, ethyl, n- and iso-propyl, n-, sec- and tert-butyl, n-, sec-, tert-pentyl and n-, sec- and tert-hexyl.

Compounds of the present invention can exist in two isomeric forms, i.e., (±)-*cis*-2,3 and (±)-*trans*-2,3. Both forms (each racemic mixtures) are within the scope of the present invention, as are the individual optical isomers e.g., (+)- or (−)-*cis*-2,3 isomers of formula I.

The compounds of this invention can be prepared according to known methods.

A. A process for the preparation of a compound of the formula I or acid addition salt thereof, which comprises reacting a compound of the formula

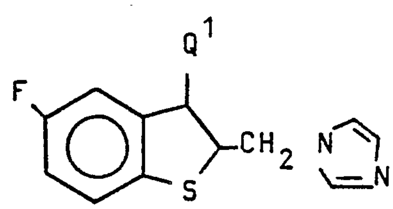

III

with a compound of the formula

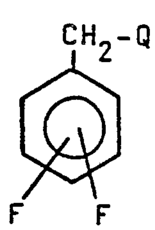

IV

wherein the fluoro substituents in the compound of formula IV are in positions 2 and 4 or 2 and 6; and one

of $Q^1$ and $Q^2$ is a hydroxy group and the other is a reactive ester group, in the presence of an inert organic solvent and a base; and the compound of the formula I is isolated as the free base or as an acid addition salt thereof.

The reactive ester group represented by $Q^1$ or more preferably by $Q^2$ can be a hydrocarbonsulfonyloxy group but is preferably a halogen atom, in particular chlorine or bromine.

The base preferably is an alkali metal base e.g., an alkali metal hydride such as sodium hydride, an alkali metal hydroxide such as potassium hydroxide or sodim hydroxide, an alkali metal amide or an alkali metal alcoholate. The organic sovlent may for example be an amide (e.g., dimethylformamide or hexamethylphosphoric acid triamide), an aromatic hydrocarbon (e.g., benzene or toluene), an ether (e.g., diethyl ether, dioxan, tetrahydrofuran or ethylene glycol dimethyl ether), a ketone (e.g., acetone) or, if the base is an alkali metal alcoholate, a $C_1$—$C_6$ alkanol (e.g., methanol or ethanol). The reaction with a halide of the formula IV can conveniently be carried out at 0—100°C, preferably at 20—60°C., e.g., at ambient temperature.

In order to increase the yield of product of formula I, the alkali metal base and the halide may be used in excess.

In a particularly preferred method of preparation of compounds of formula I, sodium hydride is added to a solution of a compound of formula III wherein $Q^1$ is a hydroxy group in dimethylformamide at 0—5°C., and, after reaction at room temperature for about an hour, the halide of formula IV (wherein $Q^2$ is a bromine or chlorine atom) is added and the reaction mixture is left for about a further hour at room temperature. The resulting compound of formula I is isolated and purified by known techniques such as extraction, chromatography, and recrystallization.

A further preferred mode of this process comprises reacting a compound of formula III with a difluorobenzyl halide in the presence of aqueous base in an organic solvent, e.g., 50% NaOH in tetrahydrofuran/water and in the presence of a suitable phase-transfer agent, or catalyst e.g., N,N,N,-tricaprylyl-N-methylammonium chloride at 0—30°C for 1—4 hrs.

Other phase-transfer catalysts useful for the invention are disclosed in Aldrichimica Acta, *9*, 35 (1976) and also U.S. Patent 3,992,432.

The product is isolated and purified in a conventional manner, e.g. by column chromatography. The most preferred compound of the present invention *cis*-2-(1H-imidazolylmethyl-3-(2′,6′-difluorobenzyloxy)-2,3-dihydro-5-fluorobenzo[b]thiophene, can be prepared in this manner from *cis*-2,3-dihydro-2-(1H-imidazolylmethyl)-3-hydroxy-5-fluorobenzo[b]thiophene and 2,6-difluorobenzyl chloride.

In general it is preferred to use as starting compound III a compound having the structure of the desired end product. Compounds of formula III can be prepared in accordance with the procedures of USP 4,352,808, 4,318,816 and 4,468,404. Compounds of formula IV can be prepared according to known methods; the difluorobenzyl halides (IV), e.g., chlorides or bromides are commercially available.

B. A process for the preparation of a compound of the formula I or acid addition salt thereof, which comprises reacting a difluorobenzyloxy-2-sulfonyloxymethylbenzo[b]thiophene (e.g., of formula V) wherein the fluoro substituents are in positions 2′ and 4′ or 2′ and 6′ with imidazole (compound of formula VI)

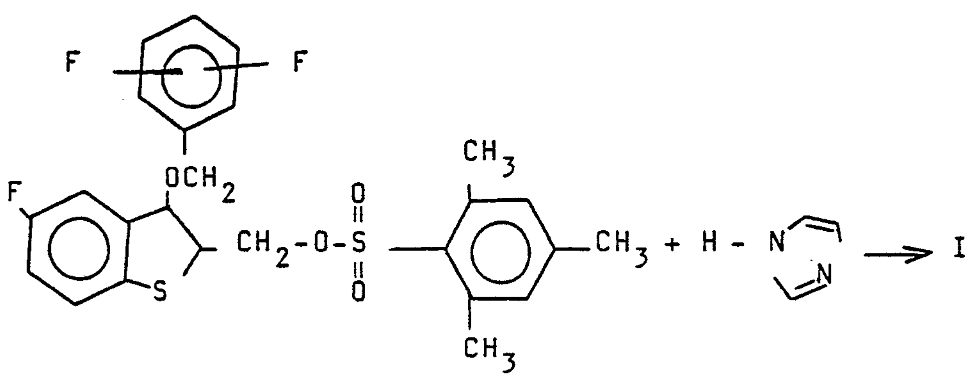

An excess of the compound (VI) is used. The reactants are refluxed in a polar solvent such as for example acetonitrile. Preferably the compound V has the steric configuration of the desired end product.

The starting compounds can be prepared according to known methods. For example the compounds of formula V can be prepared according to the following reaction scheme:

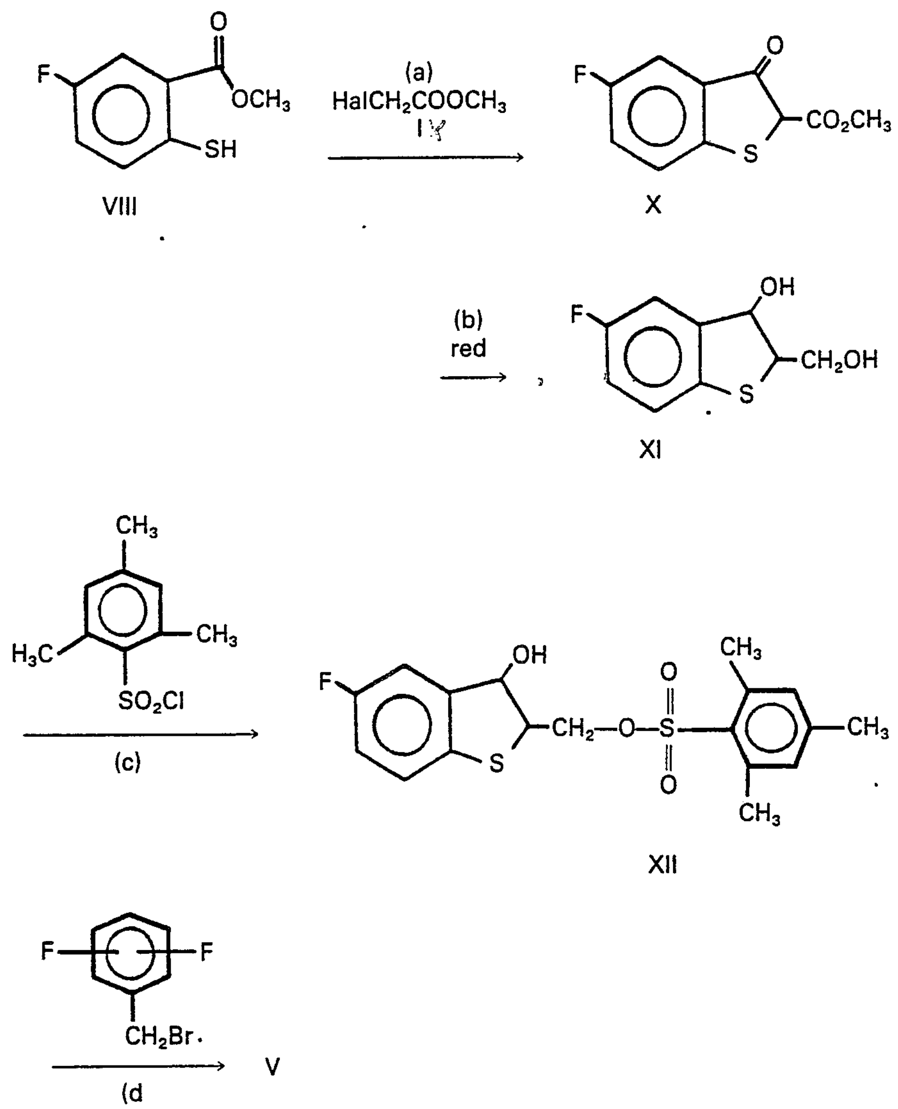


VIII

X

XI

XII

V

In formula IX Hal stands for halogen, (e.g., chloro). Reaction step (a) can be carried out in the presence of sodium in methanol. The reduction step (b) can be carried out by means of $LiAlH_4$ in an inert solvent such as tetrahydrofuran. A suitable solvent for reaction step (c) is a mixture of methylene chloride and pyridine. Reaction step (d) is carried out in a basic medium.

C. A process for the preparation of compounds of the formula I or acid addition salts thereof, which comprises reduction of the corresponding benzo[b]thiophene-1-oxide. This reduction can be carried out for example by $LiAlH_4$ in an inert solvent such as tetrahydrofuran. Preferably the starting compound has the steric configuration of the desired end product.

The starting compound can be prepared according to known methods, for example according to the following reaction scheme:

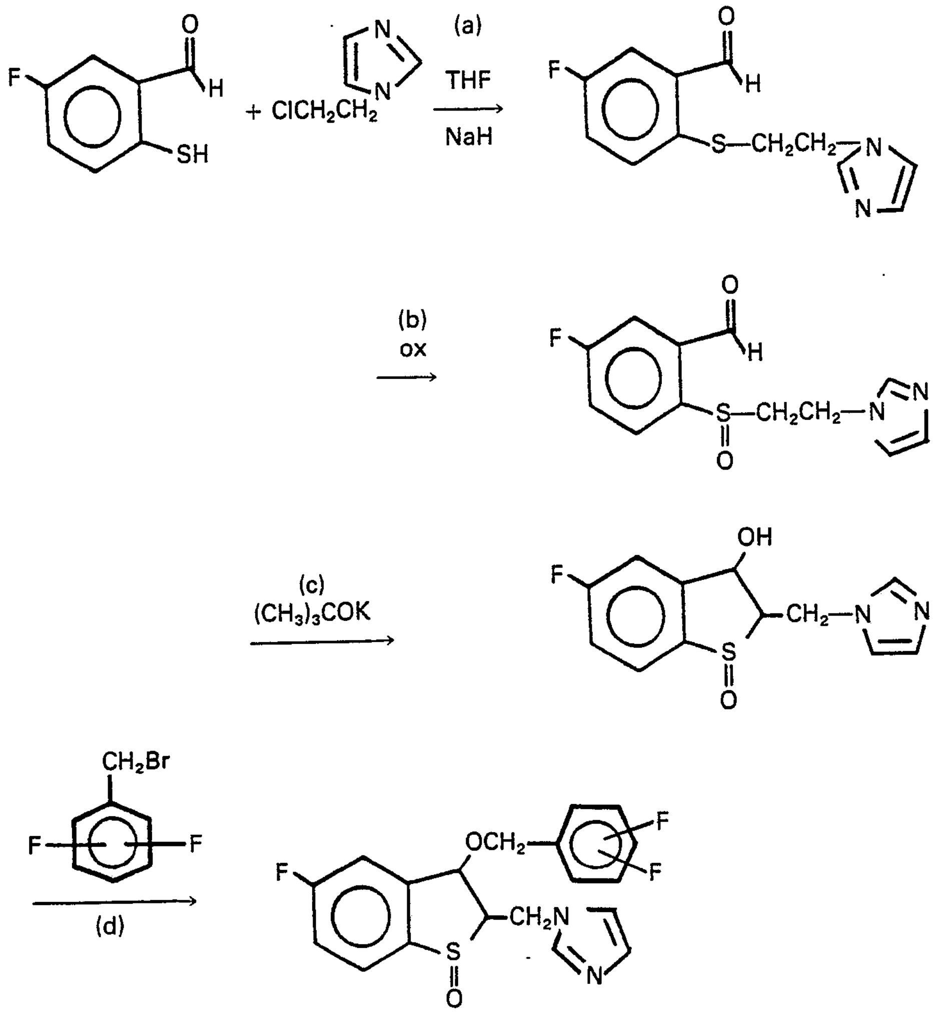


The oxidation (b) can be carried out by using m-chloroperbenzoic acid. The reaction steps (d) is carried out in a basic medium.

The product is isolated and purifed in a conventional manner, e.g., by column chromatography. The most preferred compound of the present invention, *cis*-2-(1H-imidazolylmethyl-3-(2′,6′-difluorobenzyloxy)-2,3-dihydro-5-fluorobenzo[b]thiophene, was prepared in this manner from *cis*-2,3-dihydro-2-(1H-imidazolylmethyl)-3-hydroxy-5-fluorobenzo[b]thiophene and 2,6-difluorobenzyl chloride. Compounds of formula II are prepared in accordance with the procedures of USP 4,352,808, 4,318,816 and 4,468,404. The difluorobenzyl halides, e.g., chlorides or bromides are commercially available.

The compounds of this invention exhibit broad spectrum antifungal activity, in conventional antifungal screening tests, against human and animal pathogens, such as the following: *Aspergillus*, *Candida*, *Geotrichum*, *Microsporum*, *Monosporium*, *Rhodotorula*, *Saccharomyces*, *Trichophyton*, and *Torulopsis*. In Sabourand broth medium, the most preferred compound of this invention, *cis*-2-(1H-imidazolylmethyl)-3-(2′,6′-difluorobenzyloxy)-2,3-dihdyro-5-fluorobenzo[b]thiophene had a mean Minimum Inhibitory Concentration (MIC) of 15 mcg/mL in a 48 hour test; the MIC of this compound against *Candida parapsilosis*, *Trichophyton rubrum* and *Geotrichum candidum* was <0.031 mcq/mL after 48 hours, and against *Saccharomyces cerevisiae* and *Monosporium apiospermum* was 0.125 mcg/mL after 48 hours and against various other strains of *Candida* was 32.0 mcg/mL after 48 hours. In Eagles medium, the mean MIC for the most preferred compound of this invention against six strains of *Candida* was 0.36 mcg/mL after 48 hours.

The compounds of this invention also exhibited superior topical antifungal activity in *in-vivo* tests in animals compared to miconazole and clotrimazole, present commercial antifungal products. For example, in a hamster vaginal *Candida C—60* topical infection model, the percent inhibition of infection for the most preferred compound of this invention was superior to those of miconazole and clotrimazole; in the guinea pig *Trichophyton* dermatophyte topical infection model, the percent inhibition of infection for the most preferred compound of this invention was superior to that of miconazole.

The present invention also provides a pharmaceutical composition comprising an effective antifungal amount of a compound represented by formula I or a pharmaceutically acceptable salt thereof and a pharmaceutically acceptable carried or diluent.

The preferred pharmaceutically acceptable salts are nontoxic acid addition salts formed by adding to the compound of the present invention about a stoichiometric amount of a mineral acid, such as HCl, HBr, $H_2SO_4$ or $H_3PO_4$ or of an organic acid, such as acetic, propionic, valeric, oleic, palmitic, stearic, lauric, benzoic, lactic, para-toluene sulfonic, methane sulfonic, citric, maleic, fumaric, succinic and the like, respectively.

The pharmaceutical compositions of the present invention may be formulated by combining the compound of this invention or a pharmaceutically acceptable salt thereof with any suitable, i.e., inert, pharmaceutical carrier or diluent adapted for adminstration orally, parenterally or topically in a variety of formulations. The preferred mode of administration is topical.

Examples of suitable compositions include solid or liquid compositions for oral administration such as tablets, capsules, pills, powders, granules, solutions, suspensions or emulsions. They may also be manufactured in the form of sterile solid compositions which can be dissolved in sterile water, physiological saline or some other sterile injectable medium immediately before use.

Topical dosage forms may be prepared according to procedures well known in the art, and may contain a variety of ingredients excipients and additives. The formulations for topical use include ointments, creams, lotions, powders, aerosols, pessaries and sprays. Of these, ointments, lotions and creams may contain water, oils, fats, waxes, polyesters, alcohols, or polyols, plus such other ingredients as fragrances, emulsifiers and preservatives. Powders are made by mixing the active ingredient with a readily available, inert, pulverous distributing agent, such as talcum, calcium carbonate, tricalcium phosphate, or boric acid. Aqueous suspensions of the above powders may also be made. Solutions or emulsions may also be prepared using inert solvents which are preferably nonflammable, odorless, colorless and nontoxic, for example, vegetable oils, isopropanol, dimethyl sulfoxide, hydrogenated naphthalenes, and alkylated naphthalenes. Similarly, aerosol or non-aerosol sprays may be prepared using solutions or suspensions in appropriate solvents, e.g., difluorodichloromethane, for aerosols.

Parenteral forms to be injected intravenously, intramuscularly, or subcutaneously are usually in the form of a sterile solution, and may contain salts or glucose to make the solution isotonic.

Based on the greater *in vivo* topical activity for the compound of this invention compared to micronazole, the dosage of the compound of the present invention employed to combat a given fungal infection in animals, e.g., mammals, including humans be generally somewhat less than the dosage requirements of present commercial products such as miconazole.

It will be appreciated that the actual preferred dosages of the compound of the present invention or pharmaceutically acceptable salts thereof will vary according to the particular composition formulated, the mode of application and the particular situs, host and disease being treated. Many factors that modify the action of the drug will be taken into account by the attending clinician, e.g., age, body weight, sex, diet, time of adminstration, rate or excretion, condition of the host, drug combinations reaction sensitivities and severity of the disease. Administration can be carried out continuously or periodically within the maximum tolerated dose. Optimal application rates for a given set of conditons can be readily ascertained by the attending clinician using conventinal dosage determination tests.

In general, the dosage for man ranges from about 50 mg per day to about 800 per day, with about 100 mg to 400 mg per day in single or divided doses being preferred.

The following Examples illustrate the invention.

## Examples

### Preparation

(±)-cis-2-(1H-imidazolylmethyl)-3-hydroxy-2,3-dihydro-5-fluorobenzo[b]thiophene

The procedure of Preparation 1 of USP 4,352,808, which is hereby incorporated by reference, is followed except that an equivalent quantity of 6-fluorothiocourmaran-4-one hydrochloride is substituted for 7-chloro-thiocoumaran-3-one hydrochloride to give the title compound m.p. 182—184°C.

### Example 1

(±)-cis-2-(1H-imidazolylmethyl)-3-(2′,6′-difluorobenzyloxy)-2,3-dihydro-5-fluorobenzo[b]thiophene

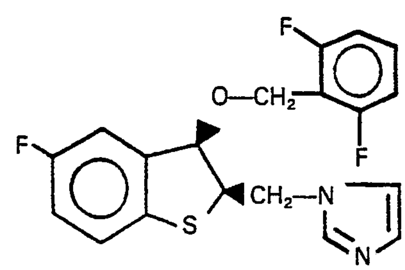

2.5 g (10 mole) of (±)-*cis*-2,3-dihydro-5-fluoro-3-hydroxy-2-(1H-imidazolylmethyl)benzo[b]thiophene,

3.10 g (15 mmole) of 2,6-difluorobenzylbromide (Aldrich Chemical Co.), and 3 drops of methyltricaprylylammonium chloride were stirred in 25 mL of 50% NaOH and 50 mL of THF at RT for 1 hr. The reaction mixture was poured into 500 mL of $HCCl_3$ with another 500 mL of $H_2O$. The $HCCl_3$ layer was dried over anhydrous $MgSO_4$ and the $CHCl_3$ removed in vacuo. The residue was chromatographed on silica gel eluting with $CHCl_3$ followed by recrystallization from cyclohexane to give 2.36 g, m.p. 167—168°, of (±)-*cis*-3-(2',6'-difluorobenzyloxy)-2,3-dihydro-5-fluoro-2-(1H-imidazolylmethyl)benzo[b]thiophene: *Calculated*: C, 60.63; H, 4.02; N, 7.44; F, 15.14; S, 8.52. *Found*: C, 60.73; H, 3.99; N, 7.17; F, 15.08; S, 8.55; $^1H$ nmr (200 MHZ, $CDCl_3$) δ: 4.04 (m, 2H, 4.49 (m, 1H), 4.85 (bs, 2H), 5.16 (d, 1H, J=5.0), 6.93 (bs, 1H), 7.05 (bs, 1H), 7.50 (bs, 1H) and 6.95—7.4 ppm (m, 6H).

Example 2

(±)-cis-2-(1H-imidazolylmethyl)-3-(2',4'-difluorobenzyloxy)-2,3-dihydro-5-fluorobenzo[b]thiophenes

The procedure of Example 1 is followed except that equivalent quantities of 2,4-difluorobenzyl bromide are substituted for 2,6-difluorobenzyl bromide to give:

(±)-*cis*-2-(1H-imidazolylmethyl)-3-(2',4'-difluorobenzyloxy)-2,3-dihydro-5-fluorobenzo[b]thiophene.

Example 3

The following are typical pharmaceutical formulations containing as the active ingredient (designated "Drug") a compound of this invention, e.g., *cis*-2-(1H-imidazolylmethyl)-3-(2',6'-difluorobenzyloxy)-2,3-dihydro-5-fluorobenzo[b]thiophene. It will be appreciated, however, that this preferred compound may be replaced by equally effective quantities of the other compounds of this invention represented by formula I.

Formulation 1

| Tablet 125.00 mg. tab. | |
|---|---|
| Drug | 125.00 mg. |
| Polyethylene glycol 6000 | 100.00 mg. |
| Sodium lauryl sulfate | 6.25 mg. |
| Corn starch | 30.00 mg. |
| Lactose, anhydrous | 87.25 mg. |
| Magnesium stearate | 1.50 mg. |

Procedure:

Heat the polyethylene glycol 6000 to 70—80°C. Mix the drug, sodium lauryl sulfate, corn starch, and lactose into the liquid and allow the mixture to cool. Pass the solidified mixture through a mill. Blend granules with magnesium stearate and compress into tablets.

Formulation 2

| Capsule 250 mg. tab. | |
|---|---|
| Drug | 250.00 mg. |
| Lactose, anhydrous | 100.00 mg. |
| Corn starch | 50.00 mg. |
| Microcrystalline cellulose | 95.00 mg. |
| Magnesium stearate | 5.00 mg. |

Procedure:

Mix the first four ingredients in a suitable mixer for 10—15 minutes. Add the magnesium stearate and mix for 1—3 minutes. Fill the mixture into suitable two-piece hard gelatin capsules using an encapsulating machine.

Example 4

(±)-cis-2-(1H-imidazolylmethyl)-3-(2',6'-difluorobenzyloxy)-2,3-dihydro-5-fluorobenzo[b]thiophene

1.85 g (0.08 mole) of sodium was dissolved in methanol (100 ml) slowly at 0—5°C. To this was added 5 g (0.026 mole) of methyl 5-fluoro-thiosalicylate and 2.9 g (0.026 mole) of methyl chloroacetate. The solution was refluxed overnight, followed by evaporation to dryness. The residue was extracted with methylene

chloride and washed with water, dried over anhydrous magnesium sulfate. The solvent was evaporated to give 5-fluoro-2,3-dihydro-2-carbomethoxybenzo(b)thiophene-3-one.

4 g (0.017 mole) of 5-fluoro-2,3-dihydro-2-carbomethoxybenzo(b)thiophene-3-one was dissolved in tetrahydrofuran (50 ml). To this was added 1.2 g (0.034 mole) of lithium aluminiumhydride. The solution was refluxed overnight, followed by evaporation of the solvent. The residue was extracted with methylene chloride, washed with water and dried over anhydrous magnesium sulphate. The solvent was evaporated to give ± *cis* and *trans*-5-fluoro-2,3-dihydro-2-hydroxymethyl-3-hydroxybenzo[b]thiophene.

To a solution of 4 g (0.02 mole) of ± *cis* and *trans*-5-fluoro-2,3-dihydro-2-hydroxymethyl-3-hydroxybenzo[b]thiophene in methylene chloride (100 ml) and pyridine (1 ml) was added 4.37 g (0.02 mole) of 2,4,6-trimethyl benzene sulfonyl chloride at 0—5°C, and the mixture was stirred at room temperature for four hours. The methylene chloride layer was separated and washed with water, dried over magnesium sulfate and solvent was evaporated to give ± *cis* and *trans*-5-fluoro-2,3-dihydro-3-hydroxy-2-[(2,4,6-trimethylbenzene)sulfonyloxymethyl]benzo[b]thiophene.

A mixture of 4 g (0.01 mole) of ± *cis* and *trans*-5-fluoro-2,3-dihydro-3-hydroxy-2-[(2,4,6-trimethylbenzene)sulfonyloxymethyl]benzo[b]thiophene, 2.07 g (0.01 mole) of 2,6-difluorobenzylbromide 50 ml of 50% aq. (W/V) sodium hydroxide and ten drops of tricaprylylmethylammonium chloride in tetrahydrofuran (100 ml) is refluxed overnight, extracted with methylene chloride and washed with water. The solvent is evaporated to give ± *cis* and *trans*-5-fluoro-3-(2′,6′-difluorobenzyloxy-2-[(2″,4″,6″-trimethylbenzene)sulfonyloxymethyl]benzo[b]thiophene.

4.92 g (0.01 mole) of ± *cis* and *trans*-5-fluoro-3-(2′,6′-difluorobenzyloxy)-2-[(2″,4″,6″-trimethylbenzene)sulfonyloxymethyl]benzo[b]thiophene and 2.04 (0.03 mole) of imidazole were refluxed in acetonitrile overnight. The reaction mixture was evaporated to dryness, the residue was chromatographed on a silica gel column eluting with methylene chloride/1% methanol to give the title compound (m.p. 167—168°C) and its transisomer.

Example 5

(±)-cis-2-(1H-imidazolylmethyl)-3-(2′,6′-difluorobenzyloxy)-2,3-dihydro-5-fluorobenzo[b]thiophene

4.68 g (0.03 mole) of 2-mercapto-5-fluorobenzaldehyde was dissolved in tetrahydrofuran (50 ml) and cooled to 0—5°C. To this was added 0.72 g (0.03 mole) of sodium hydride and stirred at 0°C for one hour. 3.91 g (0.03 mole) of chloroethyl 1N-imidazole was added to the above mixture and stirred at room temperature overnight. The mixture was evaporated to dryness, the residue was extracted with methylene chloride, washed with water and dried over magnesium sulfate. The solvent was evaporated to give 2-(1N-ethylimidazole)mercapto-5-fluorobenzaldehyde.

7.5 g (0.03 mole) of 2-(1N-ethylimidazole)mercapto-5-fluorobenzaldehyde and 5.2 g (0.03 mole) of m-chloro-perbenzoic acid were stirred in methylene chloride (100 ml) for one hour. The methylene chloride solution was washed with 5% aq. sodium bicarbonate and water. The solution was dried over magnesium sulfate and the solvent was evaporated to give 2-(1H-ethylimidazole-sulfinyl-5-fluorobenzaldehyde.

5.32 g (0.02 mole) of 2-(1N-ethylimidazole)-sulfinyl-5-fluorobenzaldehyde was dissolved in tetrahydrofuran (50 ml). To this was added 2.24 g (0.02 mole) of potassium t-butoxide and the mixture was stirred at room temperature overnight. Evaporated to dryness, extracted with methylene chloride and washed with water, dried over magnesium sulfate. Solvent evaporated to give 5-fluoro-2,3-dihydro-2-imidazolylmethyl-3-hydroxybenzo[b]thiophene-1-oxide.

7.98 g (0.03 mole) of 5-fluoro-2,3-dihydro-2-imidazolylmethyl-3-hydroxybenzo[b]thiophene-1-oxide, 6.21 g (0.03 mole) of 2,6-difluorobenzylbromide, 25 ml of 50% aq. (W/V) sodium hydroxide and ten drops of tricaprylymethylammonium chloride were stirred at room temperature in tetrahydrofuran (100 ml) overnight. After evaporation of the solvent, the residue was extracted with methylene chloride, washed with water, dried over magnesium sulfate. The solvent was evaporated to give fluoro-3-(2,6-difluorobenzyloxy)-2,3-dihydro-2-(1H-imidazolmethyl)benzo[b]thiophene-1-oxide.

A mixture of 3.92 g (0.01 mole) of 5-fluoro-3-(2,6-difluorobenzyloxy)-2,3-dihydro-2-(1H-imidazolylmethyl)benzo[b]thiophene-1-oxide and 0.38 g (0.01 mole) of lithium aluminium hydride in tetrahydrofuran (100 ml) ws refluxed overnight. After evaporation to dryness, the residue was extracted into methylene chloride and washed with water and dried over magnesium sulfate. The solvent was evaporated to give the title compound (m.p. 167—168°C).

**Claims**

1. A compound represented by the following formula

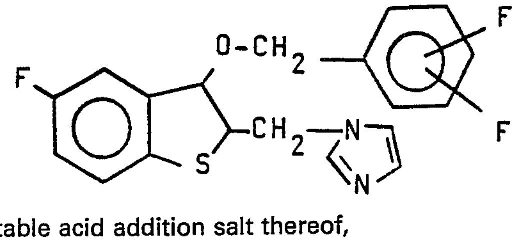

I

or a pharmaceutically acceptable acid addition salt thereof,

wherein the fluoro-substituents are in positions 2′ and 4′ or 2′ and 6′ in racemic or optically active form.

2. A compound according to claim 1 which is the *cis*-isomer.

3. A compound according to claim 1 which is (±)*cis*-2-(1H-imidazolylmethyl)-3-(2′,6′-difluorobenzyloxy)-2,3-dihydro-5-fluorobenzo[b]thiophene, (±)*cis*-2-(1H-imidazolylmethyl)-3-(2′,4′-difluorobenzyloxy)-2,3-dihydro-5-fluorobenzo[b]thiophene, or *trans*-2-(1H-imidazolylmethyl)-3-(2′,6′-difluorobenzyloxy)-2,3-dihydro-5-fluorobenzo[b]thiophene.

4. A compound according to any one of claims 1 to 3 for use in the treatment of fungal infections.

5. A pharmaceutical composition comprising an antifungally effective amount of a compound of any one of claims 1 to 3 and a pharmaceutically acceptable carrier or diluent.

6. Process for the preparation of a compound of any one of claims 1 to 3 characterized in that a compound of formula II is reacted with 2,4-difluorobenzyl halide or 2,6-difluorobenzyl halide in the presence of an aqueous base in an organic solvent and in the presence of a suitable phase-transfer agent, or catalyst

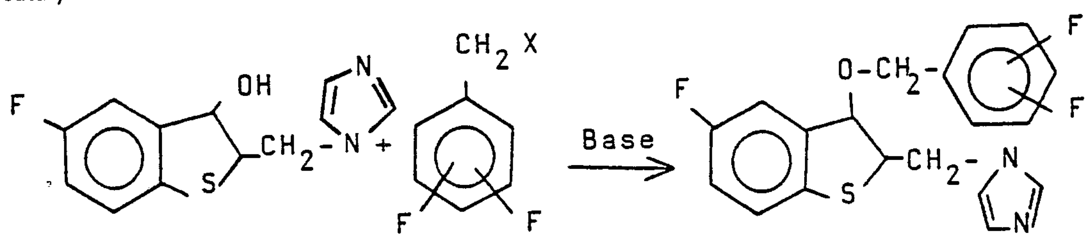


II

I

(X = par ex., Br ou Cl)

followed by isolating the product in the free form or in the form of its pharmaceutically acceptable acid addition salt.

7. Process for the preparation of a compound of any one of claims 1 to 3, characterized in that the compound is prepared by an appropriate process selected from the following processes which comprises

a) reacting a compound of the formula

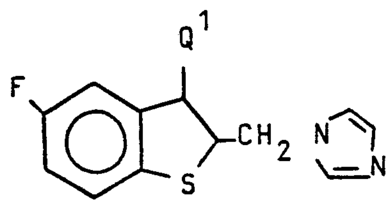

III

with a compound of the formula

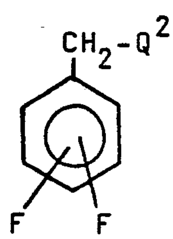

IV

wherein the fluoro-substituents are in positions 2′ and 4′ or 2′ and 6′, and one of $Q^1$ and $Q^2$ is a hydroxy group and the other is a reactive ester group, in the presence of an inert organic solvent and a base;

b) reacting a difluorobenzyloxy-2-sulfonyloxymethylbenzo[b]thiophene (V) with a compound of formula VI

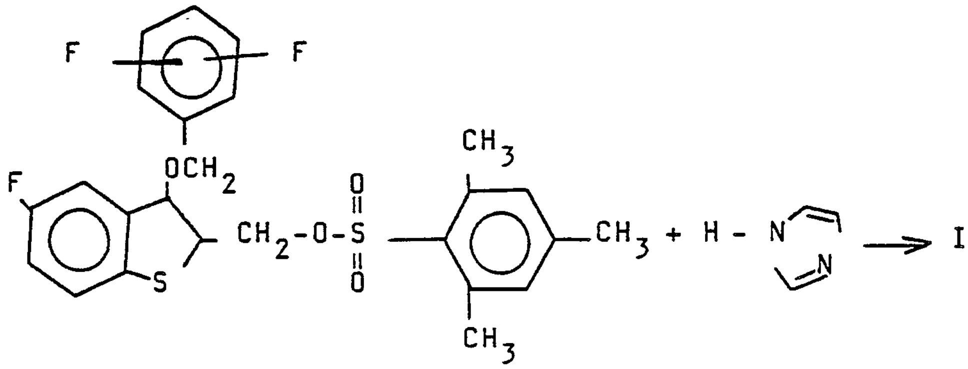

wherein the fluoro-substituents are in positions 2′ and 4′ or 2′ and 6′;

c) reduction of the corresponding benzo[b]thiophene-1-oxide

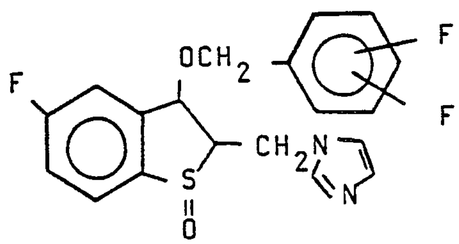

followed, if desired, by the isolation of the compound in racemic or optically active form.

**Patentansprüche**

1. Verbindung der folgenden Formel I

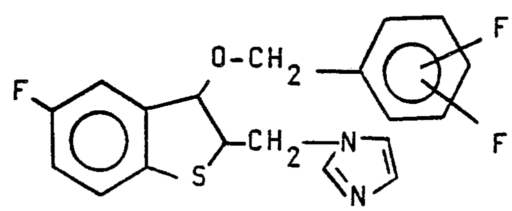

I

oder ein pharmazeutisch annehmbares Säure-Additionssalz derselben, worin die Fluor-Substituenten sich in den Positionen 2′ und 4′ oder 2′ und 6′ befinden, in racemischer oder optisch aktiver Form.

2. Verbindung nach Anspruch 1, die das cis-Isomer ist.

3. Verbindung nach Anspruch 1, die
(±)*cis*-2-(1H-Imidazolylmethyl)-3-(2′,6′-difluorbenzyloxy)-2,3-dihydro-5-fluorobenzo[b]thiophen,
(±)*cis*-2-(1H-Imidazolylmethyl)-3-(2′,4′-difluorbenzyloxy)-2,3-dihydro-5-fluorobenzo[b]thiophen oder trans-2-(1H-Imidazolylmethyl)-3-(2′,6′-difluorobenzyloxy)-2,3-dihydro-5-fluorobenzo[b]thiophen ist.

4. Verbindung nach irgendeinem der Ansprüche 1 bis 3 zur Verwendung bei der Behandlung fungaler Infektionen.

5. Pharmazeutische Zusammensetzung, umfassend eine antifungal wirksame Menge einer Verbindung nach irgendeinem der Ansprüche 1 bis 3 und ein pharmazeutisch annehmbares Trägermaterial oder Verdünnungsmittel.

6. Verfahren zur Herstellung einer Verbindung nach irgendeinem der Ansprüche 1 bis 3, dadurch gekennzeichnet, daß eine Verbindung der Formel II mit einem 2,4-Difluorobenzylhalogenid oder 2,6-Difluorobenzylhalogenid in Gegenwart einer wäßrigen Base in einem organischen Lösungsmittel und in Gegenwart eines geeigneten Phasen-Transfer-Mittels oder Katalysators umgesetzt wird

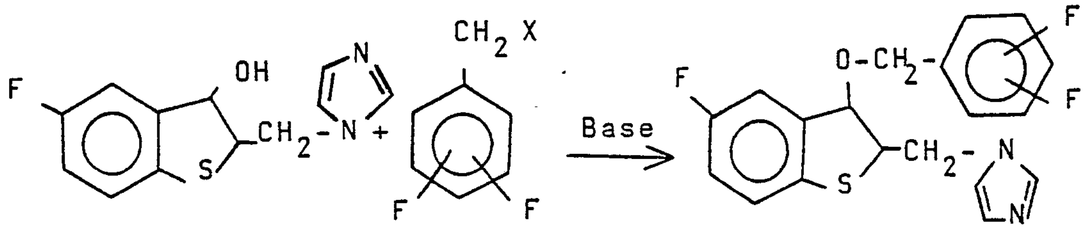


II

I

(X = par ex., Br ou Cl)

und nachfolgend das Produkt in freier Form oder Form seines pharmazeutisch annehmbaren Säure-Additionssalzes isoliert wird.

7. Verfahren zur Herstellung einer Verbindung nach irgendeinem der Ansprüche 1 bis 3, dadurch gekennzeichnet, daß die Verbindung mittels eines geeigneten Verfahrens hergestellt wird, das aus den nachstehenden Verfahren ausgewählt ist, umfassend,

a) die Umsetzung einer Verbindung der Formel

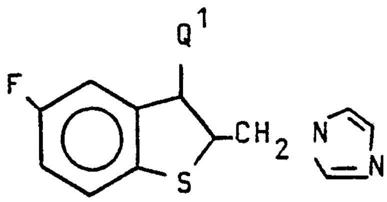

III

mit einer Verbindung der Formel

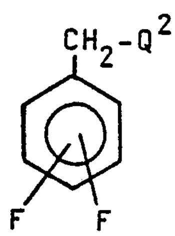

IV

worin die Fluor-Substituenten sich in den Positionen 2′ und 4′ oder 2′ und 6′ befinden und einer der Substituenten $Q^1$ und $Q^2$ eine Hydroxy-Gruppe ist und der andere eine reaktionsfähige Ester-Gruppe ist, in Gegenwart eines inerten organischen Lösungsmittels und einer Base;

b) die Umsetzung eines Difluorobenzyloxy-2-sulfonyloxymethylbenzo[b]thiophens (V) mit einer Verbindung der Formel VI

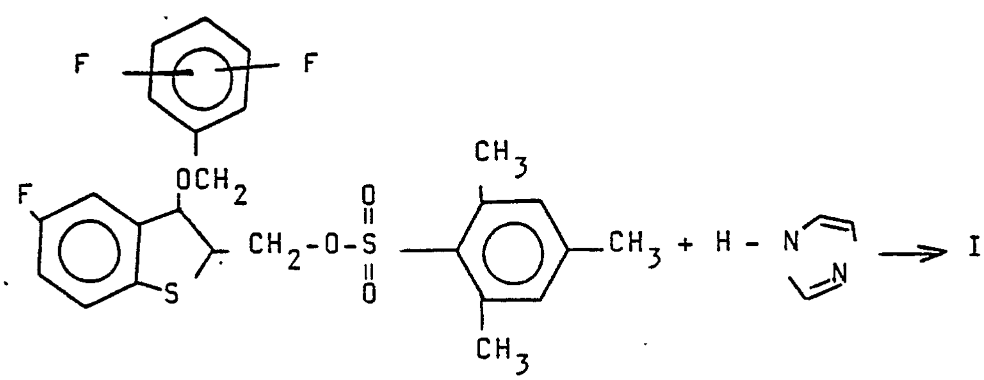

VI

worin die Fluor-Substituenten sich in den Positionen 2′ und 4′ oder 2′ und 6′ befinden;

c) die Reduktion des entsprechenden Benzo[b]thiophen-1-oxids

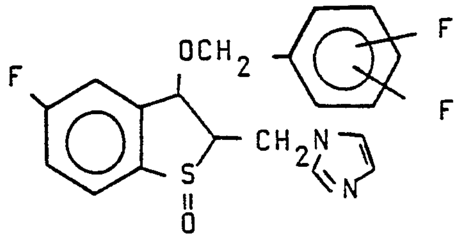

und gewünschtenfalls die nachfolgende Isolierung der Verbindung in racemischer oder optisch aktiver Form.

**Revendications**

1. Composé représenté par la formule suivante

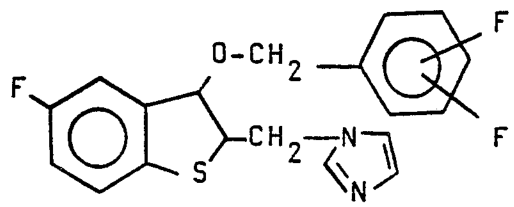

I

ou un sel d'addition d'acide de celui-ci acceptable du point de vue pharmaceutique,

dans laquelle les substituants fluoro sont aux positions 2′ et 4′ ou 2′ et 6′ sous forme racémique ou optiquement active.

2. Composé selon la revendication 1 que est l'isomère *CIS*.

3. Composé selon la revendication 1 qui est le
(±)*cis*-2-(1H-imidazolylméthyl)-3-(2′,6′-difluorobenzyloxy)-2,3-dihydro-5-fluorobenzo[b]thiophène,
(±)*cis*-2-(1H-imidazolylméthyl)-3-(2′,4′-difluorobenzyloxy)-2,3-dihydro-5-fluorobenzo[b]thiophène, ou
*trans*-2-(1H-imidazolylméthyl)-3-(2′,6′-difluorobenzyloxy)-2,3-dihydro-5-fluorobenzo(b)thiophène,

4. Composé selon l'une quelconque des revendications 1 à 3 pour une utilisation dans le traitement des infections fongiques.

5. Composition pharmaceutique comprenant une quantité efficace du point de vue antifongique d'une composé de l'une quelconque des revendications 1 à 3 et un support ou diluant acceptable du point de vue pharmaceutique.

6. Procédé pour la préparation d'un composé selon l'une quelconque des revendications 1 à 3, caractérisé en ce qu'un composé de formle II est mis à réagir avec un halogénure de 2,4-difuorobenzyle ou un halogénure de 2,6-difluorobenzyl en présence d'une base aqueuse dans un solvant organique et en présence d'un agent ou catalyseur de transfert de phase approprié

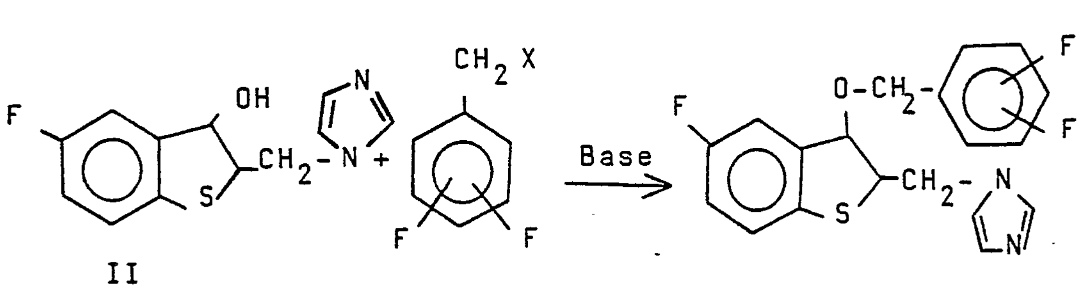


II

I

(X = par ex., Br ou Cl)

puis le produit est isolé sous forme libre ou sous forme de son sel d'addition d'acide acceptable du point de vue pharmaceutique.

7. Procédé pour la préparation d'un composé selon l'une quelconque des revendications 1 à 3, caractérisé en ce que le composé est préparé par un procédé approprié choisi parmi les procédés suivants qui comprennent

(a) la réaction d'un composé de formule

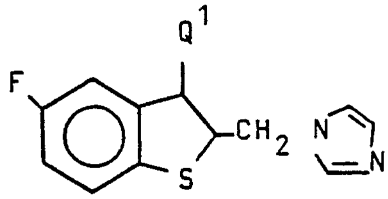

III

avec un composé de formule

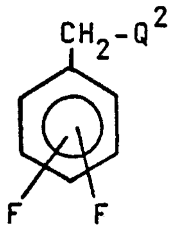

IV

dans laquelle les substituants fluoro sont aux positions 2′ et 4′ ou 2′ et 6′ et l'une des $Q^1$ et $Q^2$ est un groupe hydroxy et l'autre est un groupe ester réactif, en présence d'un solvant organique inerte et d'une base;

(b) la réaction d'un difluorobenzyloxy-2-sulfonyloxyméthylbenzo[b]thiophène (V) avec un composé de formule VI

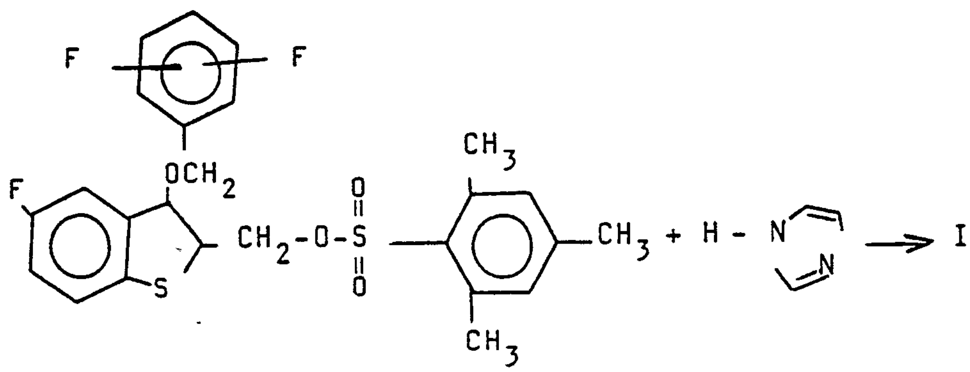

dans laquelle les substituants fluoro sont aux positions 2′ et 4′ ou 2′ et 6′;

(c) la réduction du benzo[b]thiophéne-1-oxyde correspondant

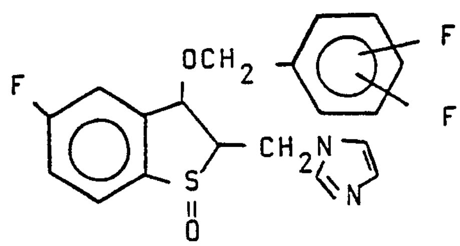

suivie si on le souhaite par l'isolement du composé sous forme racémique ou optiquement active.